# EUROPEAN PATENT APPLICATION

(11) **EP 4 803 098 A1**
(43) Date of publication of application: **09.09.2026**
(21) Application number: 24884708.9
(22) Date of filing: 29.10.2024
(51) Int. Cl.: A61K 39/395

(54) **GIPR BLOCKING TYPE ANTIBODY AND ANTIBODY CONJUGATE THEREOF**

(30) Priority: 31.10.2023 CN 202311434583; 07.03.2024 CN 202410258618; 10.10.2024 CN 202411411685
(71) Applicant: Shandong Boan Biotechnology Co., Ltd., Yantai, Shandong 264035 (CN)
(72) Inventor: DONG, Chuangchuang, Yantai, Shandong 264035 (CN); SONG, Deyong, Yantai, Shandong 264035 (CN); DOU, Changlin, Yantai, Shandong 264035 (CN); ZHANG, Junmei, Yantai, Shandong 264035 (CN); RAO, Muding, Yantai, Shandong 264035 (CN); LI, Rui, Yantai, Shandong 264035 (CN); LIU, Fang, Yantai, Shandong 264035 (CN); SHEN, Nan, Yantai, Shandong 264035 (CN); JIAO, Jie, Yantai, Shandong 264035 (CN)
(74) Representative: karo IP
(86) International application number: PCT/CN2024/127989
(87) International publication number: WO 2025/092705

(57) **Abstract**

The present disclosure provides a blocking type GIPR antibody and an antigen-binding fragment thereof, an antibody conjugate, and use thereof in the manufacture of a medicament. The GIPR antibody and the antigen-binding fragment thereof have relatively high affinity for the GIPR protein, bind to the GIPR protein expressed on cell surfaces, and block the cell-activating activity of GIP; and the GIPR antibody has good cellular internalization activity, and pharmacokinetic study shows that the GIPR antibody and the antigen-binding fragment thereof show good stability in mice, with AUC0-t of up to 15033.01 hour × µg/mL. The antibody conjugate prepared using the GIPR antibody exhibits stable and long-lasting activity in reducing the body weight of laboratory animal models and has wide application prospects.

## Description

### TECHNICAL FIELD

The present disclosure relates to the technical field of biomedicine or biopharmaceuticals, particularly to a blocking type antibody against a glucose-dependent insulinotropic polypeptide receptor (GIPR) and an antibody conjugate thereof, as well as to use of the GIPR blocking type antibody and the antibody-drug conjugate thereof in the manufacture of a medicament.

### BACKGROUND

Incretins originate from the response of intestinal endocrine cells to food intake. Incretins primarily include glucose-dependent insulinotropic polypeptide (GIP) and glucagon-like peptide-1 (GLP-1), which are produced by K cells in the proximal intestine and L cells in the distal intestine, respectively. Glucagon-like peptide-1 (GLP-1) can bind to receptors on pancreatic islet cells and stimulate insulin secretion, thereby exerting a blood glucose-lowering effect. It can also reduce food intake and delay gastric emptying, contributing to body weight control. GIP can promote fat storage and glucose-dependent insulin secretion, complementing the action of GLP-1 receptor agonists. It increases insulin secretion under hyperglycemic conditions and stimulates glucagon release under hypoglycemic conditions.

Glucose-dependent insulinotropic polypeptide (GIP) has the function of promoting adipocyte formation in both *in vitro* experiments and *in vivo* environments. The contents of free GIP in obese mice and obese human populations are high, indicating a positive correlation between GIP and an obesity-prone state.

The glucose-dependent insulinotropic polypeptide receptor (GIPR) is the receptor for the glucose-dependent insulinotropic polypeptide (GIP). Studies have confirmed that knocking out the GIPR gene in obese mice leads to reduced body weight in these mice. A global genome-related study also revealed that human populations with genotypes associated with lower GIPR activity exhibit lower body mass index (BMI). Therefore, GIPR inhibitors may represent good anti-obesity drugs.

In current research, products targeting GIP, GLP-1, or GIPR have demonstrated outstanding efficacy in weight loss and diabetes improvement. Research focused on the targets described above continues to advance. Therefore, developing GIPR blocking antibodies, as well as conjugating anti-GIPR antibodies with GLP-1 to construct dual-target conjugates targeting both GLP-1 and GIPR, holds significant importance for the development of therapeutic agents for weight loss and diabetes.

### SUMMARY

The present disclosure provides a GIPR antibody or an antigen-binding fragment thereof and an antibody conjugate thereof, as well as use of the GIPR antibody or the antigen-binding fragment thereof and the antibody conjugate in treating and/or ameliorating an abnormal metabolism disease and pharmaceutical use of the GIPR antibody or the antigen-binding fragment thereof and the antibody conjugate.

A first aspect of the present disclosure provides a GIPR antibody or an antigen-binding fragment thereof, and the antibody or the antigen-binding fragment thereof comprises 3 light chain complementarity determining regions and 3 heavy chain complementarity determining regions, wherein the 3 light chain complementarity determining regions of the antibody or the antigen-binding fragment thereof comprise an LCDR1 set forth in SEQ ID NO: 3, an LCDR2 set forth in SEQ ID NO: 4, and an LCDR3 set forth in SEQ ID NO: 5, and the 3 heavy chain complementarity determining regions of the antibody or the antigen-binding fragment thereof comprise an HCDR1 set forth in SEQ ID NO: 6, an HCDR2 set forth in SEQ ID NO: 7, and an HCDR3 set forth in SEQ ID NO: 8;
or, the 3 light chain complementarity determining regions of the antibody or the antigen-binding fragment thereof comprise an LCDR1 set forth in SEQ ID NO: 11, an LCDR2 set forth in SEQ ID NO: 12, and an LCDR3 set forth in SEQ ID NO: 13, and the 3 heavy chain complementarity determining regions of the antibody or the antigen-binding fragment thereof comprise an HCDR1 set forth in SEQ ID NO: 14, an HCDR2 set forth in SEQ ID NO: 15, and an HCDR3 set forth in SEQ ID NO: 16.

Further, the 3 light chain complementarity determining regions of the antibody or the antigen-binding fragment thereof comprise an LCDR1 set forth in SEQ ID NO: 40, an LCDR2 set forth in SEQ ID NO: 41, and an LCDR3 set forth in SEQ ID NO: 42, and the 3 heavy chain complementarity determining regions of the antibody or the antigen-binding fragment thereof comprise an HCDR1 set forth in SEQ ID NO: 43, an HCDR2 set forth in SEQ ID NO: 44, and an HCDR3 set forth in SEQ ID NO: 45.

Further, the 3 light chain complementarity determining regions of the antibody or the antigen-binding fragment thereof comprise an LCDR1 set forth in SEQ ID NO: 48, an LCDR2 set forth in SEQ ID NO: 49, and an LCDR3 set forth in SEQ ID NO: 50, and the 3 heavy chain complementarity determining regions of the antibody or the antigen-binding fragment thereof comprise an HCDR1 set forth in SEQ ID NO: 51, an HCDR2 set forth in SEQ ID NO: 52, and an HCDR3 set forth in SEQ ID NO: 53.

Further, the antibody or the antigen-binding fragment thereof comprises a light chain variable region having at least 95%, 96%, 97%, 98%, 99%, or 100% identity to the amino acid sequence set forth in SEQ ID NO: 1, and a heavy chain variable region having at least 95%, 96%, 97%, 98%, 99%, or 100% identity to the amino acid sequence set forth in SEQ ID NO: 2;
or the antibody or the antigen-binding fragment thereof comprises a light chain variable region having at least 95%, 96%, 97%, 98%, 99%, or 100% identity to the amino acid sequence set forth in SEQ ID NO: 9, and a heavy chain variable region having at least 95%, 96%, 97%, 98%, 99%, or 100% identity to the amino acid sequence set forth in SEQ ID NO: 10.

Further, the antibody or the antigen-binding fragment thereof comprises a heavy chain constant region having at least 95%, 96%, 97%, 98%, 99%, or 100% identity to the amino acid sequence set forth in SEQ ID NO: 33, and/or comprises a light chain constant region having at least 95%, 96%, 97%, 98%, 99%, or 100% identity to the amino acid sequence set forth in SEQ ID NO: 34.

Further, the antibody or the antigen-binding fragment thereof comprises a light chain variable region having at least 95%, 96%, 97%, 98%, 99%, or 100% identity to the amino acid sequence set forth in SEQ ID NO: 38, and a heavy chain variable region having at least 95%, 96%, 97%, 98%, 99%, or 100% identity to the amino acid sequence set forth in SEQ ID NO: 39.

Further, the antibody or the antigen-binding fragment thereof comprises a light chain variable region having at least 95%, 96%, 97%, 98%, 99%, or 100% identity to the amino acid sequence set forth in SEQ ID NO: 46, and a heavy chain variable region having at least 95%, 96%, 97%, 98%, 99%, or 100% identity to the amino acid sequence set forth in SEQ ID NO: 47. Further, the antibody or the antigen-binding fragment thereof is a monoclonal antibody, a polyclonal antibody, a recombinant antibody, a human antibody, a humanized antibody, a chimeric antibody, a multispecific antibody, or an antibody fragment thereof.

Further, the antibody fragment is a Fab fragment, a Fab' fragment, or a F(ab')2 fragment.

A second aspect of the present disclosure provides a nucleic acid, which encodes the anti-GIPR antibody or the antigen-binding fragment thereof described in the present disclosure.

A third aspect of the present disclosure provides a cell, which comprises the nucleic acid encoding the anti-GIPR antibody or the antigen-binding fragment thereof described in the present disclosure. A fourth aspect of the present disclosure provides a GIPR antibody conjugate, and the GIPR antibody conjugate comprises:
(a) the anti-GIPR antibody or the antigen-binding fragment thereof according to the present disclosure, and
(b) a conjugation moiety conjugated to the antibody moiety, wherein the conjugation moiety is selected from one or more of a detectable label, a chemical drug, a toxin, a radionuclide, and a short peptide.

Further, the anti-GIPR antibody or the antigen-binding fragment thereof is a monoclonal antibody, a polyclonal antibody, a recombinant antibody, a human antibody, a humanized antibody, a chimeric antibody, a multispecific antibody, or an antibody fragment thereof.

Further, the short peptide is a GLP-1 receptor agonist.

Further, the GLP-1 receptor agonist is a GLP-1 analog polypeptide.

Further, the GLP-1 analog is selected from a sequence having at least 95%, 96%, 97%, 98%, 99%, or 100% identity to an amino acid sequence set forth in any one of SEQ ID NO: 33, SEQ ID NO: 34, or SEQ ID NO: 35.

Further, the GIPR antibody conjugate further comprises (c) a linker structure; preferably, the (c) linker structure is selected from: a bromoacetyl group (abbreviated as BrAc), a structure represented by structure (I), and a structure represented by structure (II);
the structure (I) is a structure as shown below: 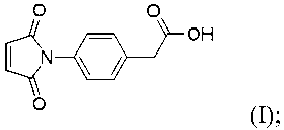
the structure represented by structure (II) is a structure as shown below:

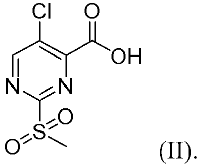

Further, the GIPR antibody conjugate has a structure shown as the following structure:

**Ab-(L-P) n**,

wherein Ab is the GIPR antibody or the antigen-binding fragment thereof provided in the present application;
L is a linker structure;
P is a GLP-1 analog polypeptide;
the subscript n is a DAR (Drug-to-Antibody Ratio) value, and n is 0-2, preferably, n is 1 or 2, and more preferably, n is 2.

Further, Ab comprises 3 light chain complementarity determining regions and 3 heavy chain complementarity determining regions, wherein the 3 light chain complementarity determining regions are an LCDR1 set forth in SEQ ID NO: 3, an LCDR2 set forth in SEQ ID NO: 4, and an LCDR3 set forth in SEQ ID NO: 5, and the 3 heavy chain complementarity determining regions of the antibody or the antigen-binding fragment thereof are an HCDR1 set forth in SEQ ID NO: 6, an HCDR2 set forth in SEQ ID NO: 7, and an HCDR3 set forth in SEQ ID NO: 8;
or, the 3 light chain complementarity determining regions of the antibody or the antigen-binding fragment thereof are an LCDR1 set forth in SEQ ID NO: 11, an LCDR2 set forth in SEQ ID NO: 12, and an LCDR3 set forth in SEQ ID NO: 13, and the 3 heavy chain complementarity determining regions of the antibody or the antigen-binding fragment thereof are an HCDR1 set forth in SEQ ID NO: 14, an HCDR2 set forth in SEQ ID NO: 15, and an HCDR3 set forth in SEQ ID NO: 16;
or the 3 light chain complementarity determining regions of the antibody or the antigen-binding fragment thereof comprise an LCDR1 set forth in SEQ ID NO: 40, an LCDR2 set forth in SEQ ID NO: 41, and an LCDR3 set forth in SEQ ID NO: 42, and the 3 heavy chain complementarity determining regions of the antibody or the antigen-binding fragment thereof comprise an HCDR1 set forth in SEQ ID NO: 43, an HCDR2 set forth in SEQ ID NO: 44, and an HCDR3 set forth in SEQ ID NO: 45;
or the 3 light chain complementarity determining regions of the antibody or the antigen-binding fragment thereof comprise an LCDR1 set forth in SEQ ID NO: 48, an LCDR2 set forth in SEQ ID NO: 49, and an LCDR3 set forth in SEQ ID NO: 50, and the 3 heavy chain complementarity determining regions of the antibody or the antigen-binding fragment thereof comprise an HCDR1 set forth in SEQ ID NO: 51, an HCDR2 set forth in SEQ ID NO: 52, and an HCDR3 set forth in SEQ ID NO: 53.

Further, the antibody or the antigen-binding fragment thereof comprises a light chain variable region having at least 95%, 96%, 97%, 98%, 99%, or 100% identity to the amino acid sequence set forth in SEQ ID NO: 1, and a heavy chain variable region having at least 95%, 96%, 97%, 98%, 99%, or 100% identity to the amino acid sequence set forth in SEQ ID NO: 2;
or the antibody or the antigen-binding fragment thereof comprises a light chain variable region having at least 95%, 96%, 97%, 98%, 99%, or 100% identity to the amino acid sequence set forth in SEQ ID NO: 9, and a heavy chain variable region having at least 95%, 96%, 97%, 98%, 99%, or 100% identity to the amino acid sequence set forth in SEQ ID NO: 10;
or the antibody or the antigen-binding fragment thereof comprises a light chain variable region having at least 95%, 96%, 97%, 98%, 99%, or 100% identity to the amino acid sequence set forth in SEQ ID NO: 38, and a heavy chain variable region having at least 95%, 96%, 97%, 98%, 99%, or 100% identity to the amino acid sequence set forth in SEQ ID NO: 39;
or the antibody or the antigen-binding fragment thereof comprises a light chain variable region having at least 95%, 96%, 97%, 98%, 99%, or 100% identity to the amino acid sequence set forth in SEQ ID NO: 46, and a heavy chain variable region having at least 95%, 96%, 97%, 98%, 99%, or 100% identity to the amino acid sequence set forth in SEQ ID NO: 47.

Further, the antibody or the antigen-binding fragment thereof comprises a heavy chain constant region having at least 95%, 96%, 97%, 98%, 99%, or 100% identity to the amino acid sequence set forth in SEQ ID NO: 33, and/or comprises a light chain constant region having at least 95%, 96%, 97%, 98%, 99%, or 100% identity to the amino acid sequence set forth in SEQ ID NO: 34.

Further, L is selected from:
a bromoacetyl group (abbreviated as BrAc), a structure represented by structure (I), and a structure represented by structure (II);
the structure (I) is a structure as shown below: 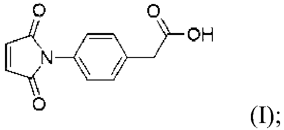
structure (II) is a structure as shown below:

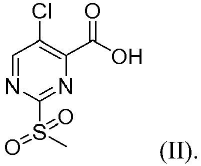

Further, P is selected from a sequence having at least 95%, 96%, 97%, 98%, 99%, or 100% identity to an amino acid sequence set forth in any one of SEQ ID NO: 35, SEQ ID NO: 36, or SEQ ID NO: 37.

Further, the amino acid at position 272, 339, or 400 of the heavy chain constant region sequence of Ab is a conjugation site.

Further, the heavy chain constant region sequence of Ab is set forth in SEQ ID NO: 33, and the amino acid at position 272, 339, or 400 of the sequence is mutated to Cys to serve as the conjugation site.

In a preferred embodiment, Ab in the structure Ab-(L-P) n of the GIPR antibody conjugate is a GIPR antibody, and the antibody comprises the heavy chain constant region set forth in SEQ ID NO: 33, the light chain constant region set forth in SEQ ID NO: 34, the heavy chain variable region set forth in SEQ ID NO: 39, and the light chain constant region set forth in SEQ ID NO: 38; the amino acid at position 339 of the heavy chain constant region sequence set forth in SEQ ID NO: 33 is mutated to Cys to serve as the conjugation site for conjugating to the (L-P) structure; L is a structure represented by structure (II), and structure (II) is a structure as shown below: 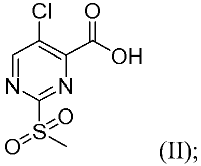 P is a sequence set forth in SEQ ID NO: 35; n is 2.

A fifth aspect of the present disclosure provides a pharmaceutical composition, which comprises the anti-GIPR antibody or the antigen-binding fragment thereof provided in the present disclosure, a nucleic acid encoding the anti-GIPR antibody or the antigen-binding fragment thereof described in the present disclosure, a cell comprising a nucleic acid encoding the anti-GIPR antibody or the antigen-binding fragment thereof described in the present disclosure, or the GIPR antibody conjugate of the present disclosure; optionally, the pharmaceutical composition further comprises a pharmaceutically acceptable excipient.

A sixth aspect of the present disclosure provides a method for treating or ameliorating an abnormal metabolism disease and/or a disease related to an abnormal metabolism disease, and the method comprises administering to a patient an effective dose of the anti-GIPR antibody or the antigen-binding fragment thereof provided in the present disclosure, a nucleic acid encoding the anti-GIPR antibody or the antigen-binding fragment thereof described in the present disclosure, a cell comprising a nucleic acid encoding the anti-GIPR antibody or the antigen-binding fragment thereof described in the present disclosure, the GIPR antibody conjugate of the present disclosure, or the pharmaceutical composition of the present disclosure.

Further, the abnormal metabolism disease includes obesity, type 2 diabetes mellitus (T2DM), and non-alcoholic fatty liver disease (NAFLD); the disease related to an abnormal metabolism disease includes obstructive sleep apnoea syndrome, chronic renal failure, heart failure, peripheral vascular disease, osteoarthritis, and cardiovascular disease (unspecified).

A seventh aspect of the present disclosure provides pharmaceutical use, and the use is use of the anti-GIPR antibody or the antigen-binding fragment thereof provided in the present disclosure, a nucleic acid encoding the anti-GIPR antibody or the antigen-binding fragment thereof described in the present disclosure, a cell comprising a nucleic acid encoding the anti-GIPR antibody or the antigen-binding fragment thereof described in the present disclosure, the GIPR antibody conjugate of the present disclosure, or the pharmaceutical composition of the present disclosure in the manufacture of a medicament for treating or ameliorating an abnormal metabolism disease and/or a disease related to an abnormal metabolism disease.

An eighth aspect of the present disclosure provides a nucleic acid construct, and the nucleic acid construct comprises a nucleic acid encoding the anti-GIPR antibody or the antigen-binding fragment thereof described in the present disclosure, and/or comprises a nucleic acid encoding a GLP-1 analog polypeptide.

A ninth aspect of the present disclosure provides a vector, and the vector comprises a nucleic acid encoding the anti-GIPR antibody or the antigen-binding fragment thereof described in the present disclosure, and/or comprises a nucleic acid encoding a GLP-1 analog polypeptide; optionally, the vector comprises other elements required for expression.

Further, the abnormal metabolism disease includes obesity, type 2 diabetes mellitus (T2DM), and non-alcoholic fatty liver disease (NAFLD); the disease related to an abnormal metabolism disease includes obstructive sleep apnoea syndrome, chronic renal failure, heart failure, peripheral vascular disease, osteoarthritis, and cardiovascular disease (unspecified).

The GIPR blocking type antibody and the antibody conjugate thereof provided in the present disclosure have one or more of the following advantages:
1. The GIPR blocking type antibody or the antigen-binding fragment thereof provided in the present disclosure has good affinity for and binding activity to the hGIPR-Fc protein, the mGIPR-Fc protein, and CHOK1-hGIPR cells expressing GIPR.
2. The GIPR blocking type antibody or the antigen-binding fragment thereof provided in the present disclosure shows a superior ability to block the activation of CHOK1-GIPR/GLP-1R cells by GIP compared to the control group.
3. The GIPR blocking type antibody or the antigen-binding fragment thereof provided in the present disclosure shows internalization activity consistent with that of the control group.
4. The GIPR blocking type antibody or the antigen-binding fragment thereof provided in the present disclosure shows a lower aggregation risk than that of the control group, which is more favorable for the development of high-concentration formulations.
5. The GIPR blocking type antibody or the antigen-binding fragment thereof provided in the present disclosure shows good pharmacokinetic activity in mice and relatively good stability in mice, with the highest AUC₀₋ₜ reaching 15033.01 hour × µg/mL.
6. The conjugates of the GIPR blocking type antibody provided in the present disclosure show relatively low hydrophobicity, so the antibody conjugates constructed in the present application are less likely to aggregate at high concentrations and are more suitable for the development of high-concentration subcutaneous formulations.
7. The conjugates of the GIPR blocking type antibody provided in the present disclosure all show excellent weight loss activity in mice, with a weight loss ratio of up to 22.35%-26.54% on day 17, and the body weight can still be lower than that of the control group for about 70 days after drug withdrawal, showing sustained weight loss activity and wide application prospects.
8. In terms of pharmacokinetics (PK), the GIPR antibody conjugates of the present disclosure demonstrate higher pharmacokinetic parameters compared with the control group, such as AUC0-t (area under the concentration-time curve of the drug *in vivo*), indicating that the GIPR antibody conjugates of the present disclosure have superior *in vivo* stability with less polypeptide dissociation.

### BRIEF DESCRIPTION OF THE DRAWINGS

FIG. 1 shows the mouse serum antibody titer in Example 1.
FIG. 2 shows the ELISA binding activity of the GIPR antibodies to the hGIPR-Fc protein in Example 3.
FIG. 3 shows the ELISA binding activity of the GIPR antibodies to the mGIPR-Fc protein in Example 3.
FIGs. 4A-4D show the binding activity of the GIPR antibodies to CHOK1-GIPR/GLP-1R cells in Example 3.
FIGs. 5A-5B show the activity of the GIPR antibodies in blocking the activation of CHOK1-GIPR/GLP-1R cells by GIP in Example 3.
FIGs. 6A-6B show the internalization activity of the GIPR antibodies on CHOK1-GIPR/GLP-1R cells in Example 3.
FIG. 7 shows the pharmacokinetic curves of the GIPR antibodies in mice in Example 4.
FIG. 8 shows the SEC chromatograms of the CE947 conjugate products in Example 5.
FIG. 9 shows a histogram of body weight inhibition rates after GIPR-GLP-1 administration in Example 6.
FIG. 10 shows the weight loss activity of conjugate products at different sites when the conjugation was performed with L1-P1 in Example 6.
FIG. 11 shows the GLP-1R activation curves of the GIPR antibody conjugate (a) and the GLP-1 polypeptides (b) in Example 7.
FIG. 12 is a diagram showing the weight loss curves in Example 8.
FIG. 13 shows the pharmacokinetic curve of the GIPR antibody conjugate in hGIPR-DIO mice in Example 9.

### DETAILED DESCRIPTION

The present disclosure will be further illustrated in conjunction with the following specific examples. The examples described herein are only some, but not all, of the examples of the present disclosure. It should be appreciated that the following examples are intended to provide those of ordinary skills in the art, to which the present disclosure pertains, a complete disclosure and description of how to utilize the methods and the compositions of the present disclosure, rather than limit the scope of the present disclosure. Based on the examples of the present disclosure, all other examples obtained by those of ordinary skills in the art without creative work shall fall within the protection scope of the present disclosure.

### Example 1. Production of blocking type GIPR Antibodies

### 1.1 Mouse immunization method

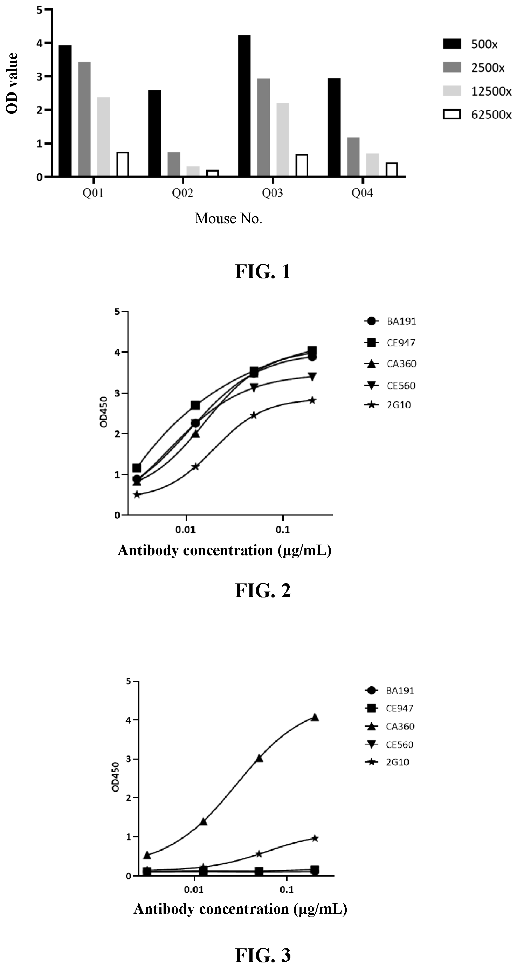

The mice used for immunization experiments were fully human antibody transgenic mice BA-huMab^{®} (self-developed by Boan). The mice were immunized with 2 types of immunogens: (1) an Fc-fused GIPR protein (GIPR corresponding to Uniprot: P48546) and (2) CHOK1 cells overexpressing GIPR (GenScript, M00486). The GIPR-Fc protein was prepared by Shandong Boan. All mice were immunized via multipoint subcutaneous injections in the abdominal and inguinal areas. The immunization doses for the GIPR-Fc protein and CHOK1/GIPR cells were 20 µg/mouse and 5 × 10⁶ cells/mouse, respectively. Complete Freund's adjuvant was used to emulsify the antigen for the first immunization, and incomplete Freund's adjuvant was used to emulsify the antigen for the subsequent immunizations; four immunizations and one booster immunization were performed. 3 days after the booster immunization, the mice were euthanized, the spleens were collected to prepare single-cell suspensions, and the single-cell suspensions were used to construct phage libraries. The detection results of the mouse serum titer are shown in FIG. 1.

### 1.2 Construction of phage libraries

The mice were sacrificed, and spleens were collected by dissection and ground to obtain splenocytes. After freezing, RNA was extracted and reverse-transcribed to obtain cDNA. Phage libraries were constructed according to conventional methods. One phage library was established for each mouse. The library capacity data for the constructed phage libraries are shown in Table 1.

**Table 1. Library capacity of constructed phage libraries**

| Library name | Q1 | Q2 | Q3 | Q4 |
|---|---|---|---|---|
| Library capacity (colony transforming units, cfu) | 1.9×10⁹ | 2.4×10⁹ | 2.3×10⁹ | 1.4×10⁹ |

### 1.3 Screening using three methods

1.3.1 Plate screening: A plate was coated with the GIPR-Fc protein. The next day, the phage libraries were added and incubated for 2 h. After washing 4-10 times, the specifically bound phages were eluted with an elution buffer.

1.3.2 Magnetic bead screening: The GIPR-Fc protein was biotinylated following the procedures of the general conjugation kit and then bound to Thermo magnetic beads. After blocking with a blocking solution, the beads were incubated with the phage libraries for 2 h. After washing 4-10 times, the specifically bound phages were eluted with an elution buffer.

1.3.3 Cell screening: CHOK1-GIPR cells were collected and incubated with the phage libraries for 2 h. After washing 4-10 times, the specifically bound phages were eluted with an elution buffer. The clones obtained by the screening and their sources are shown in Table 2.

**Table 2. Table for sources of GIPR antibody clones obtained by screening**

| Antibody No. | Source library |
|---|---|
| CE947 | Q1 |
| BA191 | Q3 |
| CA360 | Q2 |
| CE560 | Q1 |

### Example 2. Construction and Production of GIPR Antibodies

The antibody variable region genes were amplified by the conventional molecular biology technique PCR (2× Phanta Max Master Mix, Vazyme, P515-P1-AA). The antibody heavy chain variable region genes were ligated into the vector pCDNA3.4 (Life Technology) having the nucleic acid sequence of the antibody heavy chain constant region sequence via homologous recombination. The antibody light chain variable region genes were ligated into the vector pCDNA3.4 having the nucleic acid sequence of the antibody light chain constant region sequence via homologous recombination. HEK293 cells were co-transfected with plasmids extracted from the sequenced positive clones and incubated on a shaker at 37 °C\8% CO₂\125 rpm. After 7 days of the transient expression, the supernatant was purified by Protein A affinity chromatography to obtain antibodies. The antibody concentration was determined by UV280 in conjunction with the theoretical extinction coefficient.

The variable region sequences of the antibodies in the examples of the present application are shown in Table 3, and the heavy and light chain constant region sequences are shown in Table 4.

Positive clones BA191 and CE947 were constructed into IgG1 and sequenced. The light and heavy chain variable region sequences of reference antibodies 5G12 and 2G10 were from sequences SEQ ID NOs: 2025 and 2182 and SEQ ID NOs: 1958 and 2115 in a patent application with publication number CN110662558A. The variable region sequences in the amino acid sequences of each antibody are shown in Table 3 below (CDR regions are underlined, and the analysis system is the IMGT system).

**Table 3. Variable region sequences in amino acid sequences of active clones**

| Antibody ID | Light chain variable region sequence | Heavy chain variable region sequence |
|---|---|---|
| BA191 | DIQMTQSPSSLSASVGDRVTITCRASQ GISNYLAWFQQKPGKAPKSLIYAASS LQSGVPSKFSGSGSGTDFTLTISSLQP EDFATYYCQQYNSYPITFGQGTRLEI K (SEQ ID NO:1) CDRS: | EVQLVQSGAEVKKPGSSVKVSCKASGGTFS TFSSYAISWVRQAPGQGLEWMGRIIPILGIGN NAQKFQGRVTITADKSTSTAYMELSSLRSED TAVYYCAREKDWGHFDYWGQGTLVTVSS (SEQ ID NO:2) CDRS: |
| | QGISNY(SEQ ID NO:3) | GGTFSTFSSYA (SEQ ID NO:6) |
| | AAS(SEQ ID NO:4) | IIPILGIG (SEQ ID NO:7) |
| | QQYNSYPIT(SEQ ID NO:5) | AREKDWGHFDY (SEQ ID NO:8) |
| CE947 | DIQMTQSPSSLSASVGDRVTITCRASQ GISNYLAWFQQKPGKAPKSLIYAASS LQSGVPSRFSGSGSGTDFTLTISNLQP EDFATYYCLQYYDYPLTFGGGTKLEI K (SEQ ID NO:9) CDRS: | EVQLVQSGAEVKKPGSSVKVSCKASGGTFN NYAISWVRQAPGQGLEWMGRIIPFLDIASNA QKFQGKVTITADKSTSTAYMELSSLRSEDTA VYYCAREVDWGWFDSWGQGTLVTVSS (SEQ ID NO:10) CDRS: |
| | QGISNY(SEQ ID NO:11) | GGTFNNYA(SEQ ID NO:14) |
| | AAS(SEQ ID NO:12) | IIPFLDIA(SEQ ID NO:15) |
| | LQYYDYPLT(SEQ ID NO:13) | AREVDWGWFDS(SEQ ID NO:16) |
| CA360 | DIVMTQSPATLSVSPGERATLSCRASQS VSSNLAWYQQKPGQAPRLLIYGASTRA TGIPARFSGSGSGTEFTLTISSLQSEDFA VYYCQQYNNWPLTFGGGTKVEIK(SEQ ID NO:38) | EVQLVQSGAEVKKPGSSVKVSCKASGGTFSGY PINWVRQAPGQGLEWMGRIIPIPGIPNYAQKFQ DRVTITADKFANTAYMELSSLRSEDTAVYYCAR DPLGPYVFDIWGQGTMVTVSS(SEQ ID NO:39) CDRS: |
| | CDRS: | GGTFSGYP(SEQ ID NO:43) |
| | QSVSSN(SEQ ID NO:40) | IIPIPGIP(SEQ ID NO:44) |
| | GAS(SEQ ID NO:41) QQYNNWPLT(SEQ ID NO:42) | ARDPLGPYVFDI(SEQ ID NO:45) |
| CE560 | DIQMTQSPSSLSASVGDRVTITCRASQG ISSYLAWYQQKPGKAPKLLIYAASTLQ SGVPSRFSGSGSGTDFTLTISSLQPEDFA TYYCQQLNSYPLTFGGGTKVEIK(SEQ ID NO:46) CDRS: | EVQLVQSGAEVKKPGSSVKVSCKASGGTFNN YAISWVRQAPGQGLEWMGRIIPFLDIASYAQKF QGKVTITADKSTSTAYMELSSLRSEDTAVYYCA REVDWGWFDSWGQGTLVTVSS (SEQ ID NO:47) CDRS: |
| | QGISSY(SEQ ID NO:48) | GGTFNNYA(SEQ ID NO:51) |
| | AAS(SEQ ID NO:49) | IIPFLDIA(SEQ ID NO:52) |
| | QQLNSYPLT(SEQ ID NO:50) | AREVDWGWFDS(SEQ ID NO:53) |
| 5G12 | DIQLTQSPSSLSASVGDRVTITCRASQ TISRFLNWYQQKPGKAPELLIYVASS LQSGVPSRFSGSGSGTDFTLTISSLQP EDFATYYCQQSYSTLISFGQGTKLEIT (SEQ ID NO:17) CDRS: | QVQLVESGGGVVQPGRSLRLSCTASGFTFSS YGIHWVRQAPGKGLEWVAVIWYDGSNKFH ADSVKGRFTISRDNSKNTLYLQMNSLRAEDS AMYFCARGKVAGMPEAFEIWGQGTKVTVS S (SEQ ID NO:18) CDRS: |
| | QTISRF(SEQ ID NO:19) | GFTFSSYG (SEQ ID NO:22) |
| | VAS(SEQ ID NO:20) | IWYDGSNK(SEQ ID NO:23) |
| | QQSYSTLIS(SEQ ID NO:21) | ARGKVAGMPEAFEI (SEQ ID NO:24) |
| 2G10 | EIVMTQSPATLSVSPGERATLSCRASQ SVSSNLAWYQQKPGQAPRLLIYGAA TRATGIPARVSGSGSGTEFTLTISSLQS EDFAVYYCQQYNNWPLTFGGGTKVE IK (SEQ ID NO:25) | QVQLVESGGGVVQPGRSLRLSCAASGFTFSN YGMHWVRQAPGEGLEWVAAIWFDASDKY YADAVKGRFTISRDNSKNTLYLQMNSLRAE DTAVYYCARDQAIFGVVPDYWGQGTLVTVS S (SEQ ID NO:26) |
| | CDRS: | CDRS: |
| | QSVSSN (SEQ ID NO:27) | GFTFSNYG (SEQ ID NO:30) |
| | GAA (SEQ ID NO:28) | IWFDASDK (SEQ ID NO:31) |
| | QQYNNWPLT (SEQ ID NO:29) | ARDQAIFGVVPDY(SEQ ID NO:32) |

**Table 4. Heavy and light chain constant region sequences**

| Heavy chain constant region sequence: |
|---|
| ASTKGPSVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPEAAGGPSVFLFPPKPKDTLMI SRTPEVTCVVVDVSHEDPEVKFNWYVDGVEVHNAKTKPREEQYNSTYRVVSVLTVLHQDWLNGK EYKCKVSNKALAAPIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNHYTQKSLSLSPGK (SEQ ID NO:33) |

| Light chain constant region sequence: |
|---|
|  (SEQ ID NO:34) |

### Example 3. Characterization of GIPR Antibodies

### 3.1 ELISA binding activity of GIPR antibodies to human GIPR-Fc protein

Experimental groups and a control group were set. The experimental groups were BA191, CE947, CA360, and CE560, and the control group was 2G10.

The hGIPR-Fc protein was diluted to 0.2 µg/mL with a carbonate buffer and added at 100 µL/well, and the plate was coated overnight at 4 °C. The plate was blocked with 3% skim milk powder at 300 µL/well and incubated at 37 °C for 1 h. The intact antibody was subjected to a gradient dilution (starting from 0.2 µg/mL, four-fold dilution, 8 gradients) with PBST (phosphate-buffered saline + 0.05% Tween-20). The diluted antibody was added at 100 µL/well, and the plate was incubated at 37 °C for 1 h. After the plate was washed, the goat anti-human IgG (Fab specific)/HRP secondary antibody (diluted at 1:40000) (Sigma, A0293) was added at 100 µL/well, and the plate was incubated at 37 °C for 1 h. After the plate was washed, 100 µL of TMB was added to each well, and after 10 min of color development, 50 µL of 2 M H₂SO₄ was added to each well to stop the reaction. OD450 values were read on a multimode microplate reader. FIG. 2 is a graph showing the binding curves of the GIPR antibodies to hGIPR-Fc at the protein level.

As can be seen from FIG. 2, compared with the control group 2G10, the antibodies obtained by screening in the present application had superior binding sensitivity to hGIPR-Fc.

### 3.2 ELISA binding activity of GIPR antibodies to mouse GIPR-Fc protein

The mGIPR-Fc protein was diluted to 0.2 µg/mL with a carbonate buffer and added at 100 µL/well, and the plate was coated overnight at 4 °C. The plate was blocked with 3% skim milk powder at 300 µL/well and incubated at 37 °C for 1 h. The intact antibody was subjected to a gradient dilution (starting from 0.2 µg/mL, four-fold dilution, 8 gradients) with PBST (phosphate-buffered saline + 0.05% Tween-20). The diluted antibody was added at 100 µL/well, and the plate was incubated at 37 °C for 1 h. After the plate was washed, the goat anti-human IgG (Fab specific)/HRP secondary antibody (diluted at 1:40000) (Sigma, A0293) was added at 100 µL/well, and the plate was incubated at 37 °C for 1 h. After the plate was washed, 100 µL of TMB was added to each well, and after 10 min of color development, 50 µL of 2 M H₂SO₄ was added to each well to stop the reaction. OD450 values were read on a multimode microplate reader. FIG. 3 is a graph showing the binding curves of the GIPR antibodies to mGIPR-Fc at the protein level.

As can be seen from FIG. 3, the antibody CA360 obtained by screening in the present application had a relatively strong binding activity to mouse GIPR-Fc, the remaining antibodies obtained by screening had no significant binding activity to the mouse GIPR-Fc protein, and the control group 2G10 had relatively weak binding activity to mouse GIPR-Fc.

### 3.3 Binding activity of GIPR antibodies to CHOK1-GIPR/GLP-1R cells

Cell binding activity of the GIPR antibodies was performed on CHOK1-GIPR/GLP-1R cells. The antibody was subjected to a dilution (starting from 40 µg/mL, 4-fold gradient dilution, 8 concentrations) with PBS to formulate a 2× antibody solution, and the diluted antibody was added to a 96-well U-bottom plate at 50 µL/well. CHOK1-GIPR/GLP-1R cells were washed once with PBS and added to the antibody at 50 µL/well, with the cell number being 1 × 10⁵ cells/w. The plate was incubated at 4 °C for 1 h. The cells were washed twice with PBS, and then the Alexa Fluor^{®} 488-Anti-Human IgG (Jackson, 109-545-008) secondary antibody was added at 100 µL/w. The plate was incubated at 4 °C for 1 h. The cells were washed twice with PBS and then resuspended in 100 µL/w PBS, and the MFI values in the FITC channel were measured using a flow cytometer (ACEA, NovoCyte).

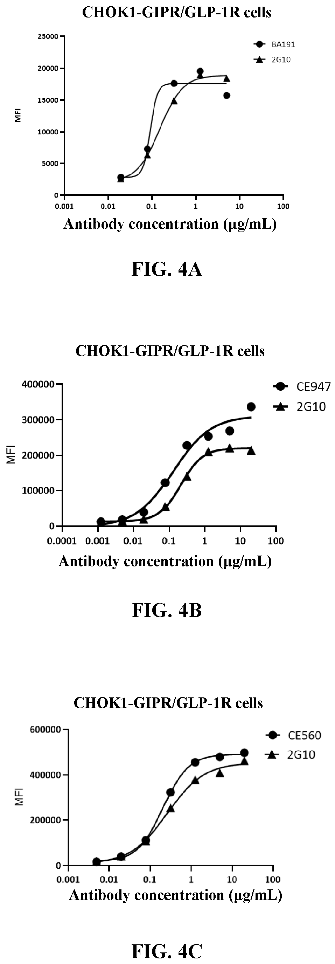

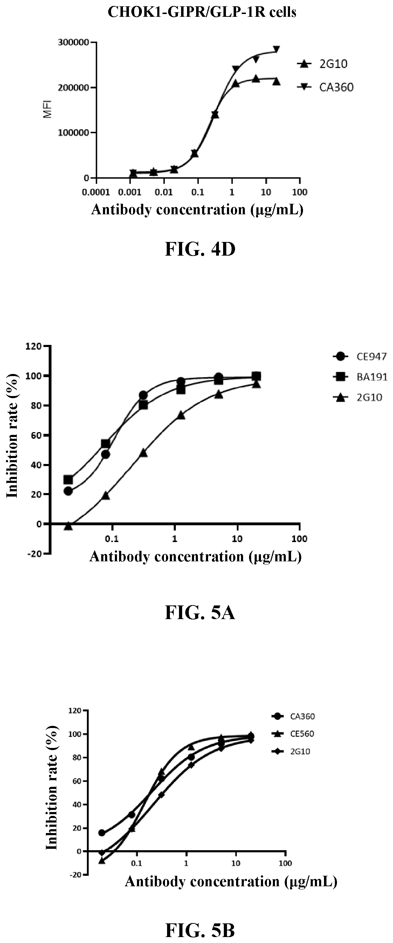

The results are shown in Table 5 and FIGs. 4A-4D. As can be seen from FIGs. 4A-4D, compared with the control group 2G10, BA191, CE947, and CE560 had higher binding activity to CHOK1-GIPR/GLP-1R cells; CA360 and 2G10 had similar EC₅₀, while the maximum binding value Rmax of CA360 was greater than that of 2G10, indicating that CA360 can bind to more GIPRs on the cell surface.

**Table 5. EC₅₀ for binding of GIPR antibodies to CHOK1-GIPR/GLP-1R cells**

| Antibody No. | EC₅₀ (µg/mL) | Maximum binding value (RFU) |
|---|---|---|
| BA191 | 0.091 | 17624 |
| 2G10 | 0.151 | 18888 |

| Antibody No. | EC₅₀ (µg/mL) | Maximum binding value (RFU) |
|---|---|---|
| CE947 | 0.131 | 311172 |
| 2G10 | 0.213 | 220451 |

| Antibody No. | EC₅₀ (µg/mL) | Maximum binding value (RFU) |
|---|---|---|
| CE560 | 0.206 | 491573 |
| 2G10 | 0.263 | 453750 |

| Antibody No. | EC₅₀ (µg/mL) | Maximum binding value (RFU) |
|---|---|---|
| CA360 | 0.328 | 280801 |
| 2G10 | 0.213 | 220451 |

### 3.4 Detection of affinity of GIPR antibodies for human GIPR protein

The binding kinetics of the GIPR antibodies to the hGIPR-His protein were measured using an Octet 96 instrument based on bio-layer interferometry (BLI) technology. The antibody was captured by a FAB2G sensor (Fortebio, 18-5125). The hGIPR-His protein (Novoprotein, C28P) was subjected to a 2-fold serial dilution starting from 50 nM with a PBST buffer to obtain 4 concentration gradients, and a 0 concentration was set for association and dissociation with the probe. The association constant (ka) and dissociation constant (kd) were calculated using a 1:1 binding model, and the equilibrium dissociation constant (KD) was calculated as the ratio of kd/ka. The results show that all candidate antibodies can bind to the corresponding antigen. The affinity data are shown in Table 6.

**Table 6. Affinity of GIPR antibodies for hGIPR-His protein**

| Antibody No. | KD (M) | Response |
|---|---|---|
| BA191 | 4.28E-09 | 0.2465 |
| CE947 | 1.13E-09 | 0.1896 |
| CE560 | 2.63E-09 | 0.2216 |
| CA360 | 3.07E-09 | 0.2459 |
| 2G10 | 1.26E-08 | 0.1421 |

A smaller KD value indicates higher affinity. The information provided in Table 6 shows that, compared with the control group 2G10, BA191, CE947, CE560, and CA360 had higher affinity for the hGIPR-His protein. The response value represents the binding signal of each antibody to the same concentration of antigen; a higher value indicates that the antibody can bind to more antigens at a steady state. Based on this parameter, BA191, CE947, CE560, and CA360 showed higher binding signals to hGIPR-His compared with the control group.

### 3.5 Neutralizing activity of GIPR antibodies at cellular level

The neutralizing activity of the GIPR antibodies at the cellular level was performed using CHOK1-GIPR/GLP-1R cells as the material. The antibody was subjected to a dilution (starting from 80 µg/mL, 4-fold gradient dilution, 8 concentrations) with the Stimulation buffer from the cAMP detection kit (purchased from Cisbio, Cat. No.: 62AM4PEB) to formulate a 4× antibody solution, which was added to a 384-well plate at 2.5 µL/well. Cells were washed once with the Stimulation buffer and then added to the antibody at 2.5 µL/well, with the cell number adjusted to 1 × 10⁴ cells/w. After 30 min of incubation at room temperature, 5 µL of GIP (Chinese Peptide (Hangzhou), GIPS-001) was added to achieve a final concentration of 0.1 µg/mL. After 30 min of incubation at 37 °C, the cAMP content was detected using the cAMP detection kit. The neutralizing activity of each GIPR antibody at the cellular level is shown in FIGs. 5A-5B and Tables 7-8.

**Table 7. IC₅₀ of GIPR antibodies BA191 and CE947 and 2G10 neutralizing activation of CHOK1-GIPR/GLP-1R cells by GIP**

| Antibody No. | IC₅₀ (µg/mL) |
|---|---|
| BA191 | 0.04 |
| CE947 | 0.05 |
| 2G10 | 0.30 |

**Table 8. IC₅₀ of GIPR antibodies CA360 and CE560 and 2G10 neutralizing activation of CHOK1-GIPR/GLP-1R cells by GIP**

| Antibody No. | IC₅₀ (µg/mL) |
|---|---|
| CA360 | 0.20 |
| CE560 | 0.14 |
| 2G10 | 0.21 |

As can be seen from FIGs. 5A-5B and Tables 7-8, the antibodies in the experimental groups numbered BA191, CE947, CE560, and CA360 all exhibited superior blocking activity at the cellular level compared with the control group 2G10.

### 3.6 Internalization activity of GIPR antibodies

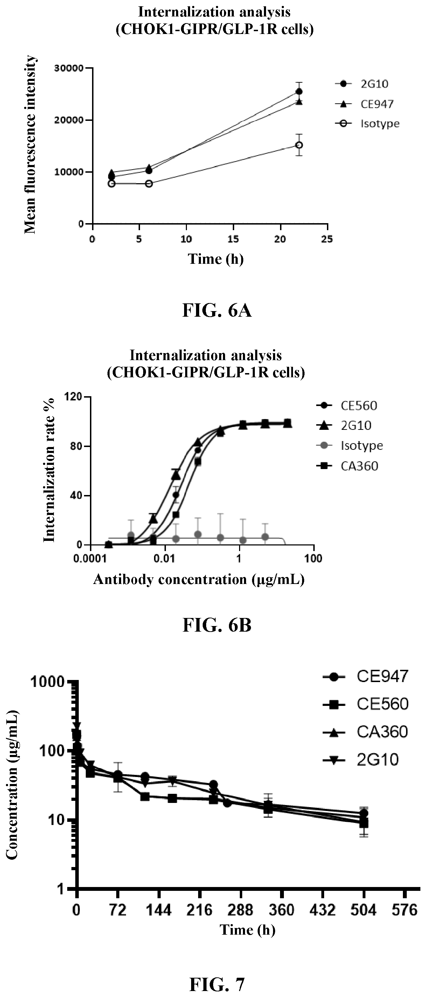

The internalization activity of the GIPR antibodies at the cellular level was performed using CHOK1-GIPR/GLP-1R cells as the material. A complete medium was formulated, and the antibody was diluted with the complete medium to achieve an antibody final concentration of 6 µg/mL. After the antibody was labeled with a labeling reagent (Invitrogen, Cat. No.: Z25611) according to the method in the instructions, cells were added at 50 µL/well. The mixture was uniformly mixed and then incubated at 37 °C for 0 h, 2 h, 6 h, and 22 h (cell number was 100000/well). The MFI values in the FITC channel were measured using a flow cytometer. Alternatively, the internalization activity of the antibody was detected using a DT3C kit, and the antibody was labeled using a DT3C labeling kit (CUSABIO, CSB-EP360556CQR1) according to the method in the instructions. The labeled antibody was subjected to a gradient dilution using a complete medium and incubated with CHOK1-GIPR/GLP-1R cells at 37 °C for 3 days, and then the cell viability was detected using a CellCounting-Lite^{®} 2.0 kit (Vazyme, Cat. No.: DD1101-01). The internalization activity of each GIPR antibody at the cellular level is shown in FIGs. 6A-6B. As shown in the figures, antibodies CE947, CE560, and CA360 exhibited internalization activity similar to that of the control group 2G10, while the irrelevant antibody group (Isotype) only showed relatively low internalization activity.

### Example 4. PK Study of GIPR Antibodies in Mice

An *in vivo* pharmacokinetic study was performed using ICR mice, and the male ICR mice were purchased from Jinan Pengyue. The mice were evenly divided into 4 experimental groups according to body weight, with 3 mice in each group, and administered at a dose of 10 mg/kg via one tail vein injection. 0.05 mL of blood was collected from the orbital venous plexus at 10 min, 1 h, 6 h, 24 h, 72 h, 120 h, 240 h, 264 h, 336 h, and 504 h after the administration of CE947 and placed in 1.5 mL EP tubes. 0.05 mL of blood was collected from the orbital venous plexus at 5 min, 1 h, 6 h, 24 h, 72 h, 120 h, 168 h, 240 h, 336 h, and 504 h after the administration of CE560, CA360, and 2G10 and placed in 1.5 mL EP tubes. The tubes were left to stand at room temperature for 1 h and then centrifuged at 8000 rpm for 10 min, and the serum was separated and stored at -80 °C before analysis.

The concentrations of the samples in mouse serum were determined by ELISA. A microplate was coated with hGIPR-Fc, which was used as a capture reagent. After blocking, standard curve samples, quality control samples, and samples to be tested were added to the microplate for incubation, during which the samples bound to the capture antigen to form antigen-antibody complexs captured on the 96-well plate. After free samples were washed away, a detection antibody (Goat Anti-Human IgG-Fab-HRP) was added to bind to the captured antigen-antibody complex on the microplate. After the free detection antibody was washed away, a substrate was added for color development, and then a stop solution was added to stop the color development reaction. The OD values were read at a wavelength of 450 nm (reference 650 nm). The sample concentration was positively correlated with the final intensity of the color developed in the reaction. The pharmacokinetic curves of the GIPR antibodies in mice are shown in FIG. 7. Table 9 shows the pharmacokinetic parameters of the GIPR antibodies CE947, CE560, and CA360 in mice, with 2G10 as the control group.

As can be seen from the pharmacokinetic curves and pharmacokinetic parameters of the GIPR antibodies in mice, the detected antibodies CE947, CE560, and CA360 all showed relatively good stability in mice, with AUC₀₋ₜ reaching 15033.01, 11339.65, and 11533.07 hour × µg/mL, respectively.

**Table 9. Pharmacokinetic parameters of GIPR antibodies in mice**

| Antibody No. | CE947 | | CE560 | | CA360 | | 2G10 | |
|---|---|---|---|---|---|---|---|---|
| Subject | mean | sd | mean | sd | mean | sd | mean | sd |
| T_{1/2} (hour) | 341.43 | 128.04 | 299.64 | 142.79 | 317.76 | 53.95 | 200.71 | 65.42 |
| Tₘₐₓ (hour) | 0.17 | 0.00 | 0.08 | 0.00 | 0.08 | 0.00 | 0.08 | 0.00 |
| Cₘₐₓ (µg/mL) | 175.39 | 15.58 | 174.70 | 10.01 | 162.48 | 23.57 | 224.22 | 8.61 |
| C₀ (µg/mL) | 189.88 | 19.82 | 181.96 | 10.63 | 168.81 | 26.91 | 233.40 | 10.59 |
| AUC₀₋ₜ (hour*µg/mL) | 15033.01 | 851.41 | 11339.65 | 150.31 | 11533.07 | 580.08 | 14700.02 | 1063.21 |
| AUC_{0-∞} (hour*µg/mL) | 21029.40 | 680.56 | 15780.76 | 3115.16 | 16546.40 | 175.32 | 17827.77 | 3036.28 |
| Vz (mL/kg) | 232.57 | 81.10 | 262.92 | 83.89 | 276.85 | 45.07 | 160.32 | 28.18 |
| Cl (mL/hour/kg) | 0.48 | 0.02 | 0.65 | 0.14 | 0.60 | 0.01 | 0.57 | 0.10 |
| MRT_{0-∞} (hour) | 418.02 | 97.94 | 392.66 | 160.84 | 419.45 | 54.46 | 276.69 | 88.34 |

### Example 5. Preparation and Analysis of GIPR Antibody Conjugates

### 5.1 Preparation of GIPR antibody conjugates

**Table 10. Linker structures used for conjugation**

| Linker No. | Linker structure |
|---|---|
| L1 | Bromoacetyl (abbreviated as: BrAc) |
| L2 | 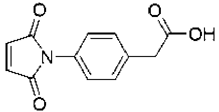 |
| L4 | 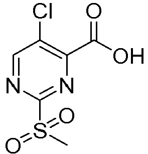 |

**Table 11. GLP-1 analog polypeptide No.**

| GLP-1 analog polypeptide No. | Sequence |
|---|---|
| P1 | HXEGTFTSDVSSYLEEQAAKEFIAWLVKGGGGGGGSGGGGSGGGGSK (SEQ ID NO: 35)-linker structure |
| P2 | HXEGTFTSDYSSYLEEQAAKEFIAWLVKGGGGGGGSGGGGSGGGGSK (SEQ ID NO: 36)-linker structure |
| P3 | HXEGTFTSDVSSYLEGQAAKEFIAWLVKGRGGGGGSGGGGSGGGGSK (SEQ ID NO: 37)-linker structure |

Where X is diaminoisobutyl (CAS Registry Number: 62-57-7).

Seven groups of GLP-1 analog polypeptide-linker structures numbered L1-P1, L2-P1, L1-P2, L2-P2, L1-P3, L2-P3, and L4-P1 were commissioned to Chinese Peptide (Hangzhou) for synthesis.

The preparation method for the GIPR antibody conjugates was as follows: The amino acid at position 272 or 339 of the heavy chain constant region sequence (set forth in SEQ ID NO: 33) of each GIPR blocking type antibody prepared in Example 2 was mutated to Cys for conjugation. The antibody was buffer-exchanged into a Tris buffer (40 mM, pH 8.2) containing 2.5 mM cystamine/2.5 mM cysteamine, incubated, and then buffer-exchanged again into a sodium acetate buffer (pH 5.2) containing sucrose. 3-8 equivalents of TCEP were added for the reaction. After the reaction was completed, the system was buffer-exchanged into a sodium phosphate buffer (pH 7.5) containing EDTA, and 8-16 equivalents of dehydroascorbic acid (DHAA) were added for oxidation for 2 h. After the oxidation was completed, when the linker was L1 or L4, the samples of the GLP-1 analog polypeptide-linker structures numbered L1-P1, L1-P2, L1-P3, or L4-P1 were added directly for conjugation; when the linker was L2, the samples of the GLP-1 analog polypeptide-linker structures numbered L2-P1, L2-P2, or L2-P3 were buffer-exchanged into a neutral PB buffer, and ring-opening was performed after the conjugation reaction was completed. The conjugate products were subjected to SEC purification using Chromdex 200 PG packing (Bestchrom, AG0083) and then buffer-exchanged into a His buffer (pH 6.0).

### 5.2 Conjugation efficiency of antibodies to linker-GLP-1 at different sites

The mutant antibody 2G10-272 (mutation at the 272 site) and the mutant antibody 2G10-339 (mutation at the 339 site) of 2G10 were constructed and produced. According to the method in Example 5.1, 2G10-272 and 2G10-339 were conjugated to L1-P2. The conjugate products were analyzed by SDS-PAGE electrophoresis, and the proportion of aggregates and the conjugation efficiency (proportion of successfully conjugated heavy chains) were calculated based on the grayscale of the electrophoretogram. The table below shows the aggregate proportion and conjugation efficiency of the conjugate products at the 2 sites. Conjugation at the 272 site produced 38% aggregates, with a conjugation efficiency of 66%. In contrast, conjugation at the 339 site produced no aggregates, and the conjugation efficiency was higher than that at the 272 site, being 79%. Therefore, conjugation at the 339 site can reduce antibody loss and improve the final yield of the conjugate product.

**Table 12. Aggregate proportion and conjugation efficiency of conjugate products at 272 and 339 sites**

| | Aggregate proportion (%) | Conjugation efficiency (%) |
|---|---|---|
| 2G10-272-L1-P2 | 38 | 66 |
| 2G10-339-L1-P2 | 0 | 79 |

### 5.3 Aggregation risk under 4 °C conditions after conjugation of antibodies to linker-GLP-1

CE947, BA191, CE560, and CA360 were conjugated to L1-P1 to obtain antibody-polypeptide conjugates. The conjugate products were stored at 4 °C for 3 days and observed for precipitate formation. After centrifugation, the protein content in the supernatant before and after storage was detected (OD280 was measured), and the reduction proportion of the conjugate products was calculated. The results are shown in Table 13 below. CA360 showed the least reduction proportion, and no aggregation occurred after storage.

**Table 13. Reduction proportion of supernatant protein after 3 days of conjugate product storage at 4 °C**

| Antibody name | Reduction proportion of supernatant protein after 3 days of storage at 4 °C |
|---|---|
| CE947 | 18% |
| BA191 | 42% |
| CE560 | 1% |
| CA360 | 0 |

### 5.4 Analysis of antibody conjugates

The Drug-to-Antibody Ratio (DAR) values of the conjugate products were analyzed using hydrophobic interaction chromatography (HIC). DAR characterizes the number of GLP-1 analog polypeptide molecules bound to the antibody. The sample was injected onto a chromatographic column (TSK-GEL Butyl-NPR, 4.6 × 35 mm, 2.5 µm) and eluted at 0.5 mL/min. The mobile phase A consisted of a 20 mM phosphate and a 25% isopropanol solution, with pH 7.0. The mobile phase B consisted of a 20 mM phosphate and a 1.5 M ammonium sulfate solution, with pH 7.0. The detection was performed at 220 nm to determine the conjugation amount.

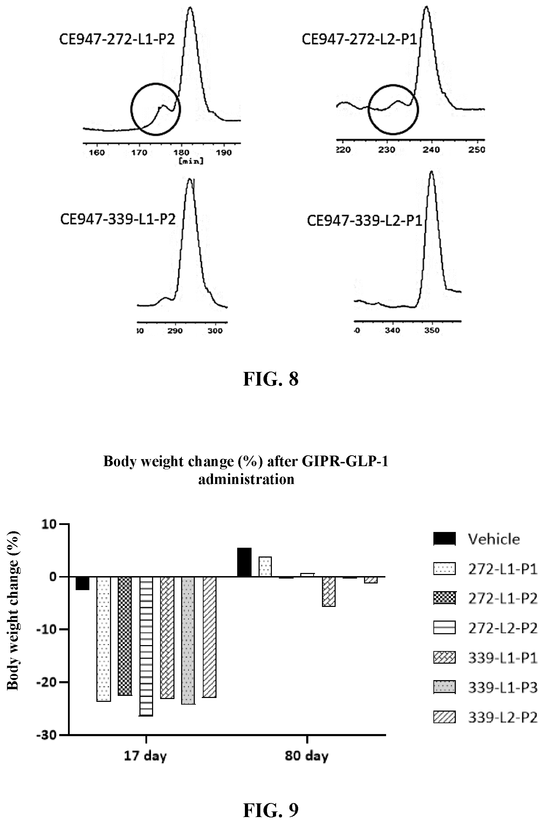

FIG. 8 shows the SEC chromatograms of the CE947 conjugate products. Table 14A records the DAR2 yields of the CE947 conjugate products after SEC purification. As can be seen from the SEC purification chromatograms of the CE947 conjugate products shown in FIG. 8, the conjugate products obtained at the 272 conjugation site showed significant aggregate peaks, whereas the conjugate products obtained at the 339 site showed essentially no or very few aggregate peaks. Therefore, after the conjugate products were subjected to SEC purification to remove aggregates, the DAR2 yields for the 339 conjugation site were significantly higher than those for the 272 site. This indicates that for the CE947 antibody, the 339 conjugation site has higher conjugation efficiency than the 272 site.

**Table 14A. DAR2 yields of CE947 conjugate products after SEC purification**

| Sample No. | DAR2 yield after SEC |
|---|---|
| CE947-339-L1-P2 | 68% |
| CE947-272-L1-P2 | 27% |
| CE947-339-L2-P1 | 55% |
| CE947-272-L2-P1 | 36% |

Table 14B is a table recording the HIC analysis retention times for the conjugates formed by the conjugation of the CA360 antibody to L1-P1, L2-P1, and L4-P1 at the 339 site and the conjugate formed by the conjugation of the 2G10 antibody to L1-P2 at the 272 site under the same HIC experimental conditions. The DAR2 HIC retention time of CA360 conjugated to the 3 types of linker-polypeptides was lower than that of 2G10.

**Table 14B. HIC analysis retention time table**

| Sample No. | Retention time min | | |
|---|---|---|---|
| | DAR0 | DAR1 | DAR2 |
| CA360-339-L1-P1 | 8 | 10.841 | 11.961 |
| CA360-339-L2-P1 | 8.1 | 10.474 | 12.086 |
| CA360-339-L4-P1 | 8.117 | 10.743 | 12.129 |
| 2G10-272-L1-P2 | 6.904 | 10.235 | 12.49 |

### Example 6. Weight Loss Activity of GIPR Antibody Conjugates in DIO Wild-Type Mice

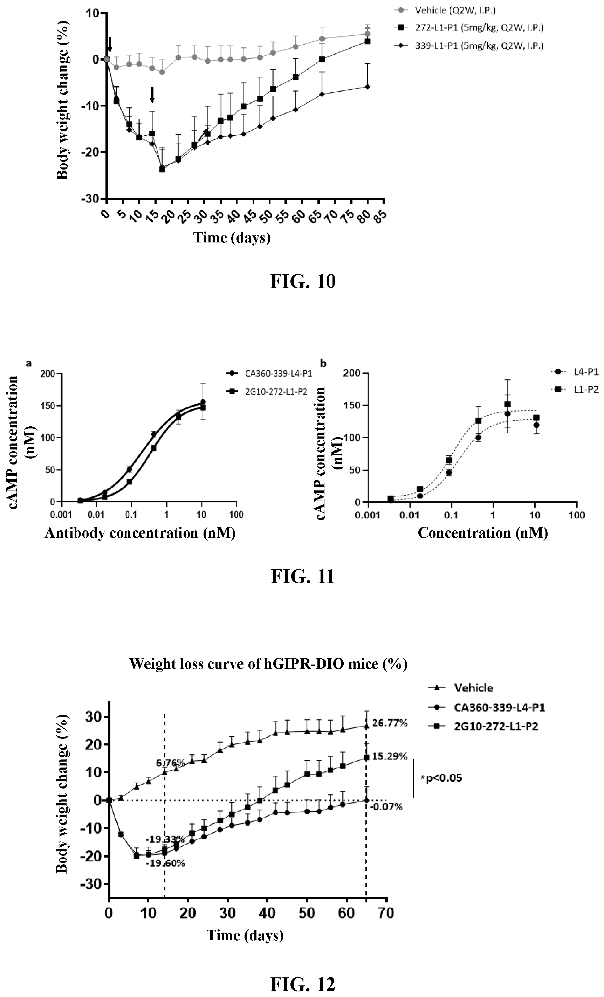

Male B-DIO mice were purchased from Biocytogen Pharmaceuticals (Beijing) Co., Ltd. (Cat. No.: 112938), with a body weight of 40-50 g. The DIO modeling super high-fat diet was purchased from Readydietech (Shenzhen) Co., Ltd. (60 kcal%, Cat. No.: D12492). B-DIO mice were housed in the SPF-level barrier facility at Shandong Boan Biotechnology Co., Ltd. Animal Feeding Center and maintained on the DIO modeling super high-fat diet. After 7 days of adaptive feeding, all mice were divided into 8 experimental groups based on body weight, including antibody-polypeptide conjugate product groups and a blank control group (Vehicle), with 6 mice per group. The antibody-polypeptide conjugate product groups were numbered according to different conjugation sites, linkers, and GLP-1 analog polypeptides as: 272-L1-P1, 272-L1-P2, 272-L2-P2, 339-L1-P1, 339-L1-P3, and 339-L2-P2. Administration started on the day of grouping. The administration dose was 5 mg/kg, the route of administration was I.P., and the administration frequency was Q2W. The body weight was measured on days 3, 7, 10, 14, 17, 22, 26, 31, 35, 38, 42, 47, 51, 58, 66, and 80 after the administration. The results were analyzed and expressed as mean ± standard error of the mean (Mean ± SEM). Table 15 shows the body weight inhibition rates after GIPR-GLP-1 administration, and FIG. 9 is a histogram generated using the values from Table 15. As can be seen from the values in Table 15 and the histogram in FIG. 9, all antibody-polypeptide conjugates of the 7 groups exhibited excellent weight loss activity in mice, achieving a weight loss rate of 22.35%-26.54% on day 17; furthermore, the body weight of the antibody-polypeptide conjugate groups remained lower than that of the control group for about 70 days after drug withdrawal, demonstrating that the antibody-polypeptide conjugate groups exhibited sustained weight loss activity. FIG. 10 shows the weight loss curves, with the arrow indicating administration. As can be seen from FIG. 10 and Table 15, when the conjugation was performed with L1-P1, the conjugate product at the 339 site showed superior weight loss activity compared with the conjugate product at the 272 site.

**Table 15. Body weight inhibition rates after GIPR-GLP-1 administration**

| | Vehicle | 272-L1-P1 | 272-L1-P2 | 272-L2-P2 | 339-L1-P1 | 339-L1-P3 | 339-L2-P2 |
|---|---|---|---|---|---|---|---|
| Inhibition rate% on day 17 | -2.72 | -23.69 | -22.65 | -26.54 | -23.24 | -24.36 | -22.95 |
| Inhibition rate% on day 80 | 5.58 | **3.92** | -0.44 | 0.71 | **-5.89** | -0.35 | -1.28 |

### Example 7. In Vitro GLP-1R Agonist Activity of GIPR Antibody Conjugate and GLP-1 Polypeptides

The GLP-1R agonist activity of the GIPR antibody conjugate at the cellular level was performed using CHOK1-GIPR/GLP-1R cells as the material. The antibody conjugate and the GLP-1 polypeptides were subjected to a gradient dilution using the Stimulation buffer from the cAMP detection kit (purchased from Cisbio, Cat. No.: 62AM4PEB) and added to a 384-well plate at 5 µL/well. CHOK1-GIPR/GLP-1R cells were washed once with the Stimulation buffer and added to the antibody at 5 µL/well, with the cell number adjusted to 5000 cells/w. After 30 min of incubation at 37 °C, the cAMP content was detected using the cAMP detection kit. FIG. 11 shows the GLP-1R activation curves of the GIPR antibody conjugate (a) and the GLP-1 polypeptides (b). Table 16 shows the EC₅₀ values of the activation curves. As can be seen from the activation curves and EC₅₀ values, L4-P1 (EC₅₀ = 0.15 nM) exhibited slightly weaker activation activity compared with L1-P2 (EC₅₀ = 0.10 nM), which is beneficial for reducing the side effects of the drug *in vivo*, while the conjugate CA360-339-L4-P1 (EC₅₀ = 0.20 nM) exhibited superior activation activity compared with 2G10-272-L1-P2 (EC₅₀ = 0.35 nM), indicating that CA360-339-L4-P1 may have a better weight loss effect *in vivo*.

**Table 16. EC₅₀ values for GLP-1R activation by GIPR antibody conjugate and GLP-1 polypeptides**

| Sample No. | EC₅₀ (nM) |
|---|---|
| CA360-339-L4-P1 | 0.20 |
| 2G10-272-L1-P2 | 0.35 |
| L4-P1 | 0.15 |
| L1-P2 | 0.10 |

### Example 8. Weight Loss Activity of GIPR Antibody Conjugate in GIPR-Humanized DIO Mice

hGIPR-DIO mice were purchased from GemPharmatech. The body weight was 40-50 g. The DIO modeling super high-fat diet was purchased from Readydietech (Shenzhen) Co., Ltd. (60 kcal%, Cat. No.: D12492). hGIPR-DIO mice were housed in the SPF-level barrier facility at Shandong Boan Biotechnology Co., Ltd. Animal Feeding Center and maintained on the DIO modeling super high-fat diet. After 7 days of adaptive feeding, all mice were divided into 5 experimental groups based on body weight, including CA360-339-L1-P1, CA360-339-L2-P1, CA360-339-L4-P1, 2G10-272-L1-P2, and a blank control group (Vehicle), with 6 mice per group. Administration started on the day of grouping. The administration dose was 5 mg/kg, the route of administration was I.P., and the administration was performed once. The body weight was measured on days 3, 7, 10, 14, 17, 21, 24, 28, 31, 35, 38, 42, 45, 50, 53, 56, 59, and 65 after the administration. The results were analyzed and expressed as mean ± standard error of the mean (Mean ± SEM).

FIG. 12 shows the weight loss curves, with the arrow indicating administration. As can be seen from this figure, CA360-339-L4-P1 showed the best weight loss effect. On day 14, the weight loss effect of CA360-339-L4-P1 (19.60% + 6.76% = 26.36%) began to show superiority over that of 2G10-272-L1-P2 (19.33% + 6.76% = 26.09%). Furthermore, 65 days after the administration, the weight loss effect of CA360-339-L4-P1 (26.77% + 0.07% = 26.84%) was significantly better than that of 2G10-272-L1-P2 (26.77% - 15.29% = 11.48%), with P < 0.05, demonstrating an excellent and sustained weight loss effect.

### Example 9. PK Study of GIPR Antibody Conjugate in hGIPR-DIO Mice

An *in vivo* pharmacokinetic study was performed using hGIPR-DIO mice purchased from GemPharmatech. The mice were evenly divided into 2 experimental groups according to body weight, with 3 mice in each group, and administered at a dose of 10 mg/kg via one tail vein injection. 0.05 mL of blood was collected from the orbital venous plexus at 10 min, 1 h, 6 h, 24 h, 96 h, 144 h, 192 h, 264 h, and 336 h after the administration and placed in 1.5 mL EP tubes. The tubes were left to stand at room temperature for 1 h and centrifuged at 8000 rpm for 10 min. The serum was separated and stored at -80 °C before analysis. The concentrations of the samples in mouse serum were determined by ELISA.

Method for detecting total antibody concentration: A microplate was coated with hGIPR-Fc, which was used as a capture reagent. After blocking, standard curve samples, quality control samples, and samples to be tested were added to the microplate for incubation, during which the samples bound to the capture antigen to form antigen-antibody complexs captured on the 96-well plate. After free samples were washed away, a detection antibody (Goat Anti-Human IgG-Fab-HRP) was added to bind to the captured antigen-antibody complex on the microplate. After the free detection antibody was washed away, a substrate was added for color development, and then a stop solution was added to stop the color development reaction. The OD values were read at a wavelength of 450 nm (reference 650 nm). The sample concentration was positively correlated with the final intensity of the color developed in the reaction.

Method for detecting concentration of conjugate still carrying polypeptide: A microplate was coated with hGIPR-Fc, which was used as a capture reagent. After blocking, standard curve samples, quality control samples, and samples to be tested were added to the microplate for incubation, during which the samples bound to the capture antigen to form antigen-antibody complexs captured on the 96-well plate. After free samples were washed away, a detection antibody (Goat Anti-Human GLP-1-HRP) was added to bind to the captured antigen-antibody complex on the microplate. After the free detection antibody was washed away, a substrate was added for color development, and then a stop solution was added to stop the color development reaction. The OD values were read at a wavelength of 450 nm (reference 650 nm). The sample concentration was positively correlated with the final intensity of the color developed in the reaction.

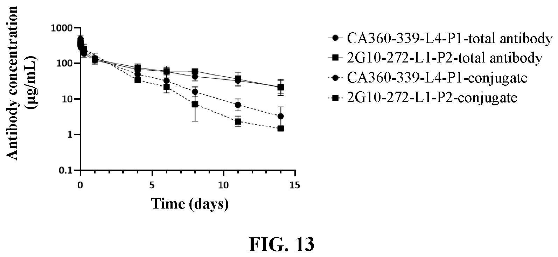

The pharmacokinetic curve of the GIPR antibody conjugate in hGIPR-DIO mice is shown in FIG. 13. Table 20 shows the pharmacokinetic parameters of the GIPR antibody conjugate CA360-339-L4-P1 and 2G10-272-L1-P2 in mice. As can be seen from the pharmacokinetic curves and pharmacokinetic parameters, the stability of the total antibody for the detected CA360-339-L4-P1 and 2G10-272-L1-P2 antibody conjugates in transgenic mice was generally consistent, with AUC₀₋ₜ reaching 23152.24 and 23538.48, respectively. However, the stability of the CA360-339-L4-P1 conjugate was higher than that of 2G10-272-L1-P2, with AUC₀₋ₜ reaching 16963.40 and 15514.44, respectively, indicating less polypeptide dissociation for the CA360-339-L4-P1 conjugate in transgenic mice.

**Table 17. PK parameters of GIPR antibody conjugate in hGIPR-DIO mice**

| Parameter | CA360-339-L4-P1-total antibody | | 2G10-272-L1-P2-total antibody | | CA360-339-L4-P1-conjugate | | 2G10-272-L1-P2-conjugate | |
|---|---|---|---|---|---|---|---|---|
| | **mean** | **SD** | **mean** | **SD** | **mean** | **SD** | **mean** | **SD** |
| T_{1/2} (hour) | 169.39 | 26.85 | 109.09 | 55.77 | 61.25 | 8.99 | 37.76 | 6.18 |
| Tₘₐₓ (hour) | 0.17 | 0.00 | 0.17 | 0.00 | 0.17 | 0.00 | 0.17 | 0.00 |
| Cₘₐₓ (µg/mL) | 408.43 | 99.34 | 345.13 | 32.58 | 552.93 | 116.49 | 382.75 | 19.04 |
| C₀ (µg/mL) | 429.86 | 105.44 | 356.05 | 42.68 | 578.35 | 121.78 | 401.66 | 21.48 |
| AUC₀₋ₜ (hour*µg/mL) | 23152.24 | 3787.52 | 23538.48 | 4414.17 | 16963.40 | 1736.32 | 15514.44 | 3720.40 |
| AUC_{0-∞} (hour*µg/mL) | 29996.45 | 6592.85 | 27522.94 | 7964.95 | 17539.90 | 1956.59 | 15604.78 | 3721.38 |
| Vz (mL/kg) | 81.90 | 6.50 | 55.26 | 13.24 | 51.00 | 11.32 | 35.23 | 2.69 |
| Cl (mL/hour/kg) | 0.34 | 0.10 | 0.38 | 0.11 | 0.57 | 0.07 | 0.66 | 0.16 |
| MRT_{0-∞} (hour) | 224.16 | 43.84 | 168.68 | 50.30 | 81.42 | 11.83 | 55.07 | 9.71 |

## Claims

1. A GIPR antibody or an antigen-binding fragment thereof, comprising 3 light chain complementarity determining regions and 3 heavy chain complementarity determining regions, wherein the 3 light chain complementarity determining regions of the antibody or the antigen-binding fragment thereof comprise an LCDR1 set forth in SEQ ID NO: 40, an LCDR2 set forth in SEQ ID NO: 41, and an LCDR3 set forth in SEQ ID NO: 42, and the 3 heavy chain complementarity determining regions of the antibody or the antigen-binding fragment thereof comprise an HCDR1 set forth in SEQ ID NO: 43, an HCDR2 set forth in SEQ ID NO: 44, and an HCDR3 set forth in SEQ ID NO: 45;
the 3 light chain complementarity determining regions of the antibody or the antigen-binding fragment thereof comprise an LCDR1 set forth in SEQ ID NO: 3, an LCDR2 set forth in SEQ ID NO: 4, and an LCDR3 set forth in SEQ ID NO: 5, and the 3 heavy chain complementarity determining regions of the antibody or the antigen-binding fragment thereof comprise an HCDR1 set forth in SEQ ID NO: 6, an HCDR2 set forth in SEQ ID NO: 7, and an HCDR3 set forth in SEQ ID NO: 8;
the 3 light chain complementarity determining regions of the antibody or the antigen-binding fragment thereof comprise an LCDR1 set forth in SEQ ID NO: 11, an LCDR2 set forth in SEQ ID NO: 12, and an LCDR3 set forth in SEQ ID NO: 13, and the 3 heavy chain complementarity determining regions of the antibody or the antigen-binding fragment thereof comprise an HCDR1 set forth in SEQ ID NO: 14, an HCDR2 set forth in SEQ ID NO: 15, and an HCDR3 set forth in SEQ ID NO: 16; or
the 3 light chain complementarity determining regions of the antibody or the antigen-binding fragment thereof comprise an LCDR1 set forth in SEQ ID NO: 48, an LCDR2 set forth in SEQ ID NO: 49, and an LCDR3 set forth in SEQ ID NO: 50, and the 3 heavy chain complementarity determining regions of the antibody or the antigen-binding fragment thereof comprise an HCDR1 set forth in SEQ ID NO: 51, an HCDR2 set forth in SEQ ID NO: 52, and an HCDR3 set forth in SEQ ID NO: 53.

2. The antibody or the antigen-binding fragment thereof according to claim 1, comprising a light chain variable region having at least 95%, 96%, 97%, 98%, 99%, or 100% identity to the amino acid sequence set forth in SEQ ID NO: 38, and a heavy chain variable region having at least 95%, 96%, 97%, 98%, 99%, or 100% identity to the amino acid sequence set forth in SEQ ID NO: 39;
comprising a light chain variable region having at least 95%, 96%, 97%, 98%, 99%, or 100% identity to the amino acid sequence set forth in SEQ ID NO: 1, and a heavy chain variable region having at least 95%, 96%, 97%, 98%, 99%, or 100% identity to the amino acid sequence set forth in SEQ ID NO: 2;
comprising a light chain variable region having at least 95%, 96%, 97%, 98%, 99%, or 100% identity to the amino acid sequence set forth in SEQ ID NO: 9, and a heavy chain variable region having at least 95%, 96%, 97%, 98%, 99%, or 100% identity to the amino acid sequence set forth in SEQ ID NO: 10; or
comprising a light chain variable region having at least 95%, 96%, 97%, 98%, 99%, or 100% identity to the amino acid sequence set forth in SEQ ID NO: 46, and a heavy chain variable region having at least 95%, 96%, 97%, 98%, 99%, or 100% identity to the amino acid sequence set forth in SEQ ID NO: 47.

3. The antibody or the antigen-binding fragment thereof according to claim 1 or 2, comprising a heavy chain constant region having at least 95%, 96%, 97%, 98%, 99%, or 100% identity to the amino acid sequence set forth in SEQ ID NO: 33, and/or comprising a light chain constant region having at least 95%, 96%, 97%, 98%, 99%, or 100% identity to the amino acid sequence set forth in SEQ ID NO: 34.

4. A nucleic acid, wherein the nucleic acid encodes the anti-GIPR antibody or the antigen-binding fragment thereof according to any one of claims 1-3.

5. A cell, comprising the nucleic acid according to claim 4.

6. A GIPR antibody conjugate, wherein the GIPR antibody conjugate has a structure as shown below:
Ab-(L-P) n,
wherein Ab is the anti-GIPR antibody or the antigen-binding fragment thereof according to any one of claims 1-3;
L is a linker structure;
P is a conjugation moiety conjugated to the GIPR antibody or the antigen-binding fragment thereof;
the subscript n is a DAR (Drug-to-Antibody Ratio) value, and n is 0-2, preferably, n is 1 or 2, and more preferably, n is 2;
P is selected from: one or more of a detectable label, a chemical drug, a toxin, a radionuclide, and a short peptide;
preferably, P is a GLP-1 analog polypeptide;
preferably, the GLP-1 receptor agonist is a GLP-1 analog;
preferably, the GLP-1 analog is selected from a sequence having at least 95%, 96%, 97%, 98%, 99%, or 100% identity to an amino acid sequence set forth in any one of SEQ ID NO: 35, SEQ ID NO: 36, or SEQ ID NO: 37.

7. The GIPR antibody conjugate according to claim 6, wherein the linker structure is selected from:
a) a bromoacetyl group;
b) a structure represented by structure (I); or
c) a structure represented by structure (II);
the structure (I) is a structure as shown below: 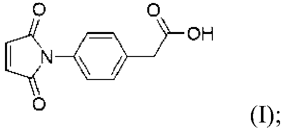
the structure represented by structure (II) is a structure as shown below: 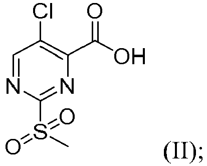
preferably, an amino acid at position 272, 339, or 400 of the heavy chain constant region sequence of Ab is a conjugation site;
more preferably, the heavy chain constant region sequence of Ab is set forth in SEQ ID NO: 33, and an amino acid at position 272, 339, or 400 of the sequence set forth in SEQ ID NO: 33 is mutated to Cys to serve as the conjugation site.

8. A pharmaceutical composition, comprising the anti-GIPR antibody or the antigen-binding fragment thereof according to any one of claims 1-3, the nucleic acid according to claim 4, the cell according to claim 5, or the GIPR antibody conjugate according to any one of claim 6 or 7, wherein
optionally, the pharmaceutical composition further comprises a pharmaceutically acceptable excipient.

9. Use of the anti-GIPR antibody or the antigen-binding fragment thereof according to any one of claims 1-3, the nucleic acid according to claim 4, the cell according to claim 5, the GIPR antibody conjugate according to any one of claim 6 or 7, or the pharmaceutical composition according to claim 8 in the manufacture of a medicament for treating or ameliorating an abnormal metabolism disease and a disease related to an abnormal metabolism disease.

10. The use according to claim 9, wherein the abnormal metabolism disease comprises obesity, type 2 diabetes mellitus, and non-alcoholic fatty liver disease; the disease related to an abnormal metabolism disease comprises obstructive sleep apnoea syndrome, chronic renal failure, heart failure, peripheral vascular disease, osteoarthritis, and cardiovascular disease.
